# EUROPEAN PATENT APPLICATION

(11) **EP 4 539 060 A1**
(43) Date of publication of application: **16.04.2025**
(21) Application number: 23822908.2
(22) Date of filing: 26.05.2023
(51) Int. Cl.: G16H 40/63

(54) **PROGRAMMED CONTROL DEVICE, PROGRAMMED CONTROL SYSTEM, ELECTRONIC DEVICE AND COMPUTER-READABLE STORAGE MEDIUM**

(30) Priority: 17.06.2022 CN 202210689123
(71) Applicant: Sceneray Co., Ltd, Suzhou, Jiangsu 215123 (CN)
(72) Inventor: ZHOU, Guoxin, Suzhou, Jiangsu 215000 (CN); DENG, Ze, Suzhou, Jiangsu 215000 (CN); ZHANG, Jun, Suzhou, Jiangsu 215000 (CN)
(74) Representative: Cabinet Beaumont
(86) International application number: PCT/CN2023/096656
(87) International publication number: WO 2023/241338

(57) **Abstract**

Provided are a programmed control device, a programmed control system, an electronic device and a computer-readable storage medium. The programmed control device is configured to: receive a pre-acquisition operation of a user; in response to the pre-acquisition operation, control a bioelectric acquisition device to enter a pre-acquisition mode to acquire a first bioelectric signal of a patient; in response to the first bioelectric signal not meeting a preset condition, generate first prompt information and send the first prompt information to user equipment, and/or stop pre-acquisition; in response to the first bioelectric signal meeting a preset condition, receive an acquisition confirmation operation of the user; and in response to the acquisition confirmation operation, control the bioelectric acquisition device to enter a formal acquisition mode to acquire a second bioelectric signal of the patient. The patient is provided with an opportunity to adapt to an acquisition process in advance, thereby ensuring the life safety of the patient.

## Description

This application claims priority to Chinese Patent Application No. 202210689123.5 filed Jun. 17, 2022, the disclosure of which is incorporated herein by reference in its entirety.

### TECHNICAL FIELD

The present application relates to the technical field of implantable medical devices, for example, a programmed control device, a programmed control system, an electronic device and a computer-readable storage medium.

### BACKGROUND

In an existing programmed control system, as long as a data acquisition instruction is issued, a patient stimulator will exit a programmed control mode and enter an acquisition mode. Once the patient stimulator enters the acquisition mode, the only thing to do is to wait until the stimulator ends the acquisition, which is a long and irreversible process. In this case, no matter whether an acquired signal meets a requirement, the acquisition cannot be stopped, an acquisition parameter cannot be reset, and the acquisition cannot be performed again. Moreover, when a patient suffers from a physical discomfort or a symptomatic episode, the stimulator cannot exit the acquisition and cannot switch back to the programmed control mode, and a stimulation parameter cannot be adjusted, resulting in a relatively serious safety hazard.

Patent CN114082102A discloses a closed-loop vagus nerve stimulator based on a scalp electroencephalogram signal. The closed-loop vagus nerve stimulator based on the scalp electroencephalogram signal includes a signal acquisition module, a scalp electroencephalogram signal processing module and a vagus nerve stimulator. The signal acquisition module is connected to the scalp electroencephalogram signal processing module. The signal acquisition module is used for acquiring a scalp electroencephalogram signal. The scalp electroencephalogram signal processing module is used for processing the scalp electroencephalogram signal, determining whether epilepsy occurs according to the processed scalp electroencephalogram signal, and transmitting an epilepsy occurrence signal to the vagus nerve stimulator. The vagus nerve stimulator is used for generating a stimulation pulse after receiving the signal. In this manner, the signal acquisition module still needs to perform long-term acquisition. Once the acquisition begins, the only thing to do is to wait until the acquisition ends. An acquisition process cannot be intervened, and the life safety of a patient cannot be ensured.

Therefore, there is an urgent need to provide a programmed control device, a programmed control system, an electronic device and a computer-readable storage medium to solve the problems existing in the related art.

### SUMMARY

An object of the present application is to provide a programmed control device and a programmed control system, thereby ensuring that a doctor acquires ideal acquired data, providing a patient with an opportunity to adapt to an acquisition process in advance and ensuring the life safety of the patient.

The object of the present application is achieved through the technical solutions described below.

In a first aspect, the present application provides a programmed control device. The programmed control device is configured to perform the operations described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

In a possible implementation, the preset condition includes at least one of the conditions described below.

A validity score of the first bioelectric signal is not less than a preset validity score threshold.

The first bioelectric signal is not abnormal.

In a possible implementation, the programmed control device is configured to acquire the validity score of the first bioelectric signal in the manners described below.

The first bioelectric signal is sampled to obtain multiple sampling points.

For each of the multiple sampling points, a comparison value between an amplitude of each of the multiple sampling points and a preset amplitude is calculated one by one, and a sampling point of the multiple sampling points whose comparison value is not less than a preset comparison threshold is used as a valid sampling point.

The validity score of the first bioelectric signal is determined according to the number of valid sampling points.

In a possible implementation, the programmed control device is configured to detect whether the first bioelectric signal is abnormal in the manners described below.

The first bioelectric signal is input into an anomaly detection model to output a predicted similarity corresponding to the first bioelectric signal, where the anomaly detection model is configured to compare the first bioelectric signal with each abnormal bioelectric signal in an anomaly database to obtain a similarity between the first bioelectric signal and each abnormal bioelectric signal, and a highest similarity among multiple similarities corresponding to the first bioelectric signal is used as the predicted similarity corresponding to the first bioelectric signal.

In response to the predicted similarity being not less than a preset similarity threshold, that the first bioelectric signal is abnormal is determined.

In response to the predicted similarity being less than a preset similarity threshold, that the first bioelectric signal is not abnormal is determined.

In a possible implementation, the preset condition includes: a validity score of the first bioelectric signal is not less than a preset validity score threshold.

In response to the first bioelectric signal not meeting the preset condition, the programmed control device is further configured to perform the operations described below.

Acquisition parameter configuration information corresponding to the bioelectric acquisition device is acquired according to the first bioelectric signal, and the bioelectric acquisition device is controlled to reenter the pre-acquisition mode.

In a possible implementation, the first bioelectric signal includes an electroencephalogram signal, and after controlling the bioelectric acquisition device to enter the pre-acquisition mode, the programmed control device is further configured to perform the operations described below.

A pain score of the patient is acquired according to the first bioelectric signal.

In response to the pain score being not less than a preset pain score threshold, the pre-acquisition is stopped.

In a possible implementation, the programmed control device is further configured to acquire the pain score of the patient in the manner described below.

The first bioelectric signal is input into a pain prediction model to obtain the pain score of the patient.

A training process of the pain prediction model includes the steps described below.

A first training set is acquired, where the first training set includes multiple first training data, and each of the multiple first training data includes a bioelectric signal of a sample object and annotated data of a pain score of the sample object.

For each of the multiple first training data in the first training set, the processing described below is performed.

The bioelectric signal of the sample object in each of the multiple first training data is input into a preset first deep learning model to obtain predicted data of the pain score of the sample object.

A model parameter of the first deep learning model is updated according to the predicted data and the annotated data of the pain score of the sample object.

Whether a preset first training termination condition is met is detected. In response to the preset first training termination condition being met, the trained first deep learning model is used as the pain prediction model. In response to the preset first training termination condition not being met, the first deep learning model is continuously trained with the next first training data of the multiple first training data.

In a possible implementation, the bioelectric acquisition device is a stimulator implanted into the patient, and the programmed control device is further configured to perform the operation described below.

Stimulation parameter configuration information corresponding to the patient is acquired according to the second bioelectric signal after the formal acquisition is completed with the bioelectric acquisition device so that the bioelectric acquisition device applies corresponding electrical stimulation energy to the patient.

In a second aspect, the present application provides a programmed control system. The programmed control system includes a programmed control device and a bioelectric acquisition device.

The programmed control device is communicatively connected to the bioelectric acquisition device and is configured to perform the operations described below.

A pre-acquisition operation of a user is received.

In response to the pre-acquisition operation, the bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

In a third aspect, the present application provides an electronic device. The electronic device includes a memory and a processor, where the memory stores a computer program, and the processor, when executing the computer program, implements the steps described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

In a fourth aspect, the present application provides a computer-readable storage medium storing a computer program, where the computer program, when executed by a processor, implements the steps described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

Using the programmed control device, the programmed control system, the electronic device and the computer-readable storage medium provided in the present application has at least the advantages described below.

The pre-acquisition provides the doctor with an opportunity to remodify an acquisition parameter and reissue an acquisition instruction. If the pre-acquired bioelectric signal does not reach a standard (with poor quality and an abnormality), the acquisition parameter can be readjusted over and over again (the pre-acquisition has a short acquisition duration, and compared with the formal acquisition, the pre-acquisition has less trial and error time and cost), thereby ensuring that the doctor acquires the ideal acquired data. The pre-acquisition provides the patient with the opportunity to adapt to the acquisition process in advance, thereby ensuring the life safety of the patient.

### BRIEF DESCRIPTION OF DRAWINGS

The present application is further described below in conjunction with drawings and embodiments.
FIG. 1 is a flowchart of a method for controlling a programmed control device according to an embodiment of the present application.
FIG. 2 is a flowchart for acquiring a validity score according to an embodiment of the present application.
FIG. 3 is a flowchart for detecting whether an abnormality has occurred according to an embodiment of the present application.
FIG. 4 is a portion of a flowchart of a method for controlling a programmed control device according to an embodiment of the present application.
FIG. 5 is a block diagram of a programmed control system 300 according to an embodiment of the present application.

### DETAILED DESCRIPTION

The present application is further described below in conjunction with the drawings and the embodiments. It is to be noted that under the premise of no conflict, the embodiments or the technical features described below may be arbitrarily combined to form a new embodiment.

An application field (an implantable neurostimulator) of the present application is briefly described below.

An implantable neurostimulation system (a neurostimulation system) mainly includes a stimulator implanted *in vivo* (an implantable neurostimulator which is a type of neurostimulator) and a programmed control device disposed in a patient *in vitro.* In an existing neuromodulation technology, an electrode is mainly implanted into a particular structure (a target site) *in vivo* through a stereotactic surgery, and an electrical pulse is sent by the stimulator implanted into the patient to the target site via the electrode to regulate electrical activity and functions of a corresponding neural structure and a network, thereby improving a symptom and alleviating a disease pain. The stimulator may be any one of an implantable nerve electrical stimulation apparatus, an implantable cardiac electrical stimulation system (also referred to as a cardiac pacemaker), an implantable drug delivery system (IDDS) and a lead switching apparatus. The implanted nerve electrical stimulation apparatus is, for example, a deep brain stimulation (DBS) system, an implantable cortical nerve stimulation (CNS) system, an implantable spinal cord stimulation (SCS) system, an implantable sacral nerve stimulation (SNS) system or an implantable vagus nerve stimulation (VNS) system.

The stimulator may include an implantable pulse generator (IPG), an extended lead and an electrode lead. Disposed in the patient *in vivo*, the IPG provides controllable electrical stimulation energy to a living tissue depending on a sealed cell and a circuit and provides one or two controllable particular electrical stimulation energy to a particular region of the living tissue via the implanted extended lead and electrode lead. The extended lead is used together with the IPG. As a transmission medium of an electrical stimulation signal, the extended lead transmits the electrical stimulation signal generated by the IPG to the electrode lead. The electrode lead releases electrical stimulation energy to the particular region of the living tissue via multiple electrode contacts. One or more electrode leads are disposed on one side or two sides of the implantable medical device. Multiple electrode contacts are disposed on the electrode lead and may be evenly or non-evenly arranged in a circumferential direction of the electrode lead. As an example, the electrode contacts are arranged in the circumferential direction of the electrode lead in an array of four rows and three columns (12 electrode contacts in total). The electrode contacts may include stimulation electrode contacts and/or acquisition electrode contacts. The electrode contact may be, for example, in a shape of a sheet, a ring or a dot.

In some possible embodiments, the stimulated living tissue may be a brain tissue of the patient, and the stimulated part may be a particular part of the brain tissue. When disease types of patients are different, stimulated parts are generally different, and the number of used stimulation contacts (single source or multi-source), the application of one or more (single channel or multi-channel) particular electrical stimulation signals and stimulation parameter data are also different. The applicable disease types are not limited in the present application and may be disease types applicable to deep brain stimulation (DBS), spinal cord stimulation (SCS), pelvic stimulation, gastric stimulation, periphery nerve stimulation and functional electrical stimulation. Disease types that may be treated or managed by DBS include, but are not limited to, seizure disorders (for example, epilepsy), pain, migraine, mental illnesses (for example, major depressive disorder (MDD)), bipolar disorder, anxiety disorder, post-traumatic stress disorder, dysthymia, obsessive-compulsive disorder (OCD), behavior disorder, mood disorder, memory disorder, mental state disorder, movement disorders (for example, essential tremor or Parkinson's disease), Huntington's disease, Alzheimer's disease, drug addiction, autism or other neurological or psychiatric disorders and impairment. When DBS is used for treating patients suffered from drug addiction, DBS can help drug addicts rehabilitate drugs, thereby improving the sense of happiness and quality of life of the drug users.

Using a deep brain stimulator (DBS) as an example, the stimulator in the present application is illustrated. When a programmed control connection is established between the programmed control device and the stimulator, the programmed control device may be used for adjusting stimulation parameters of an electrical stimulation signal of the stimulator, or the stimulator may sense a bioelectric activity of a deep brain of the patient, and the adjustment of the stimulation parameters of the electrical stimulation signal of the stimulator may be continued by using the sensed bioelectric activity.

The stimulation parameter of the electrical stimulation signal may include one or more of a frequency (for example, the number of electrical stimulation pulse signals within a unit time of 1 s, in Hz), a pulse width (the duration of each pulse, in µs), an amplitude (generally represented by voltage, that is, the strength of each pulse, in V), a timing (for example, may be continuation or burst), a stimulation mode (including one or more of a current mode, a voltage mode, a timing stimulation mode and a cyclic stimulation mode), doctor-controlled upper and lower limits (a range that can be adjusted by a doctor) and patient-controlled upper and lower limits (a range that can be autonomously adjusted by a patient). In a specific application, each stimulation parameter of the stimulator may be adjusted in the current mode or the voltage mode.

The programmed control device may be a doctor programmer (a programmer used by the doctor) or a patient programmer (a programmer used by the patient). The doctor programmer may be, for example, an intelligent terminal device equipped with programmed control software, such as a tablet computer, a notebook computer, a desktop computer or a mobile phone. The patient programmer may be, for example, an intelligent terminal device equipped with programmed control software, such as a tablet computer, a notebook computer, a desktop computer or a mobile phone, and may also be another electronic device (for example, a charger or data acquisition device with a programmed control function) with a programmed control function.

The data interaction between the doctor programmer and the stimulator is not limited in the present application. When the doctor performs remote programmed control, the doctor programmer can perform data interaction with the stimulator via a server and the patient programmer. When the doctor performs offline programmed control with the patient face to face, the doctor programmer can perform data interaction with the stimulator via the patient programmer and can also directly perform data interaction with the stimulator.

The patient programmer may include a host (in communication with the server) and a slave (in communication with the stimulator), and the host can be communicatively connected to the slave. The doctor programmer can perform data interaction with the server via a 3G/4G/5G network. The server can perform data interaction with the host via the 3G/4G/5G network. The host can perform data interaction with the slave via a Bluetooth protocol/WIFI protocol/USB protocol. The slave can perform data interaction with the stimulator via an operating frequency band of 401-406 MHz/operating frequency band of 2.4-2.48 GHz. The doctor programmer can directly perform data interaction with the stimulator via the operating frequency band of 401-406 MHz/operating frequency band of 2.4-2.48 GHz.

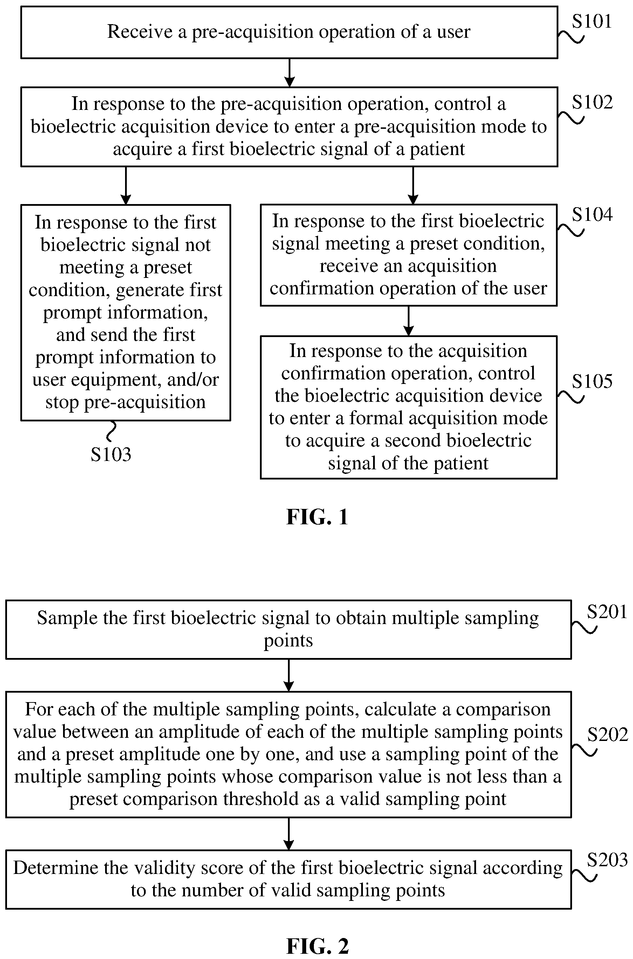

Referring to FIG. 1, FIG. 1 is a flowchart of a method for controlling a programmed control device according to an embodiment of the present application.

An embodiment of the present application provides a method for controlling a programmed control device. The method includes steps S101 to S105.

In step S101, a pre-acquisition operation of a user is received.

In step S102, in response to the pre-acquisition operation, a bioelectric acquisition device is controlled to enter a pre-acquisition mode to acquire a first bioelectric signal of a patient.

In step S103, in response to the first bioelectric signal not meeting a preset condition, first prompt information is generated and sent to user equipment, and/or pre-acquisition is stopped.

In step S104, in response to the first bioelectric signal meeting a preset condition, an acquisition confirmation operation of the user is received.

In step S105, in response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

In this manner, the bioelectric acquisition device may have two acquisition modes, which are a pre-acquisition mode and a formal acquisition mode, respectively. In the pre-acquisition mode, the acquisition duration is relatively short and may be, for example, 2 minutes, 5 minutes or 10 minutes, and an acquisition process can be stopped at any time. In the formal acquisition mode, the acquisition duration is very long and may be, for example, 1 hour, 2 hours or 4 hours, and an acquisition process cannot be stopped at any time. Before entering the long-term formal acquisition mode, the bioelectric acquisition device must enter the pre-acquisition mode to obtain the first bioelectric signal through the pre-acquisition, and whether the first bioelectric signal meets the preset condition is determined. On the one hand, if the preset condition is not met, the pre-acquisition process is immediately stopped, and the subsequent formal acquisition cannot be performed, and/or the user is prompted that the first bioelectric signal does not meet the preset condition, thereby facilitating the user taking a corresponding measure. For example, the user may reset an acquisition parameter for pre-acquisition again. Alternatively, the user may also not perform the acquisition temporarily by switching the programmer to a programmed control mode and may acquire the bioelectric signal of the patient another day. On the other hand, if the preset condition is met, the user confirms whether the formal acquisition is performed, and the bioelectric acquisition device can enter the formal acquisition mode to acquire the second bioelectric signal through the formal acquisition as long as the user confirms that a physical state of the patient has no problem. The relatively long acquisition duration corresponding to the second bioelectric signal is very helpful for analyzing a condition of the patient.

In the present application, the pre-acquisition provides a doctor with an opportunity to remodify the acquisition parameter and reissue an acquisition instruction. If the pre-acquired bioelectric signal does not reach a standard (with poor quality and an abnormality), the acquisition parameter can be readjusted over and over again (the pre-acquisition has a short acquisition duration, and compared with the formal acquisition, the pre-acquisition has less trial and error time and cost), thereby ensuring that the doctor acquires ideal acquired data (the ideal acquired data means, for example, that a waveform of the acquired signal is a waveform expected by the doctor or an average amplitude of the acquired signal reaches an expectation value of the doctor). The pre-acquisition provides the patient with an opportunity to adapt to the acquisition process in advance, thereby ensuring the life safety of the patient.

The user may be a patient, or may be a person who takes care of the patient, such as a doctor, a nurse, a caregiver, a nanny or a family member of the patient.

The user equipment is not limited in the present application and may be a mobile phone, tablet computer, desktop computer, notebook computer or smart wearable device used by the user.

The bioelectric acquisition device is not limited in the present application and may include any one or more of an electroencephalogram acquisition device (for example, an electrode cap, a cortical electrode or an intracranial electrode), an electrocardiogram acquisition device, an electrooculogram acquisition device and an electromyogram acquisition device. Correspondingly, the first bioelectric signal may include any one or more of an electroencephalogram signal, an electrocardiogram signal, an electrooculogram signal and an electromyogram signal.

In some possible embodiments, the bioelectric acquisition device may be the stimulator, the first bioelectric signal may include an electroencephalogram signal, and the bioelectric acquisition device has both functions of sensing the electroencephalogram signal and releasing electrical stimulation energy.

When the preset condition is not met, only the pre-acquisition may be stopped, only the first prompt information may be sent, or the pre-acquisition may be stopped and the first prompt information may be sent.

The first prompt information may be in a form of character, video or voice and may be, for example, text information that "the first bioelectric signal is abnormal" or "the first bioelectric signal is too weak, and it is recommended to re-acquire the first bioelectric signal".

In some possible embodiments, when the preset condition is not met, the pre-acquisition may be stopped, and the prompt information may be generated and sent to the user equipment.

In some other possible embodiments, when the preset condition is not met, the prompt information may be generated and sent to the user equipment, and the pre-acquisition may be stopped.

In some possible embodiments, when the preset condition is met, second prompt information may be generated and sent to the user equipment.

The second prompt information may be in the form of character, video or voice and may be, for example, voice information that "the first bioelectric signal reaches a standard, and it is recommended to start the formal acquisition".

In some possible embodiments, the preset condition includes at least one of the conditions described below.

A validity score of the first bioelectric signal is not less than a preset validity score threshold.

The first bioelectric signal is not abnormal.

In this manner, on the one hand, the preset condition may be that the validity score of the first bioelectric signal is not less than the preset validity score threshold. When the preset condition is met, it indicates that the signal strength of the first bioelectric signal is relatively high and the subsequent formal acquisition can be performed under the same acquisition parameter, thereby facilitating analyzing the condition of the patient. When the preset condition is not met, it indicates that the signal strength of the first bioelectric signal is relatively low and the current acquisition parameter is inappropriate. On the other hand, the preset condition may be that the first bioelectric signal is not abnormal. When the preset condition is met, it indicates that the patient does not suffer from a symptomatic episode and the subsequent formal acquisition can be performed. When the preset condition is not met, it indicates that the patient is more likely to suffer from the attack, an acquisition work is suspended and an electrical stimulation therapy needs to be performed.

In some possible embodiments, the preset condition may include: the validity score of the first bioelectric signal is not less than the preset validity score threshold, and the first bioelectric signal is not abnormal.

The validity score may be represented by a number, for example, 60, 80 or 90. The higher the value is, the higher the validity score is. The validity score may also be represented by a letter, for example, A, B, C or D. The higher the ranking of the letter is, the higher the validity score is.

The preset validity score threshold is not limited in the present application and is, for example, 70, 80 or 85.

Referring to FIG. 2, FIG. 2 is a flowchart for acquiring a validity score according to an embodiment of the present application.

In some possible embodiments, a process of acquiring the validity score of the first bioelectric signal may include steps S201 to S203.

In step S201, the first bioelectric signal is sampled to obtain multiple sampling points.

In step S202, for each of the multiple sampling points, a comparison value between an amplitude of each of the multiple sampling points and a preset amplitude is calculated one by one, and a sampling point of the multiple sampling points whose comparison value is not less than a preset comparison threshold is used as a valid sampling point.

In step S203, the validity score of the first bioelectric signal is determined according to the number of valid sampling points.

In this manner, the validity score can be determined in the manner of sampling and detecting the first bioelectric signal. The first bioelectric signal is discretized through the sampling to obtain the multiple sampling points. The number of valid sampling points can reflect the validity score of the entire first bioelectric signal. In this manner, only a limited number of sampling points need to be analyzed instead of analyzing the entire first bioelectric signal, thereby significantly reducing a calculation amount and improving detection efficiency.

The comparison value between the amplitude of the sampling point and the preset amplitude may be obtained through difference calculation or ratio calculation.

The preset amplitude and the preset comparison threshold are not limited in the present application. The preset amplitude may be 10 µV, 20 µV or 300 µV. The preset comparison threshold may be 1 (µV), 0.2 (µV) or 0.5 (µV).

In some possible embodiments, determining the validity score of the first bioelectric signal according to the number of valid sampling points may include the step described below.

The validity score of the first bioelectric signal is determined according to the number of valid sampling points and the (total) number of multiple sampling points.

Specifically, the validity score may be a ratio of the number of valid sampling points to the total number of sampling points.

In some other embodiments, determining the validity score of the first bioelectric signal according to the number of valid sampling points may include the steps described below.

When the number of valid sampling points is between 100 and 200, the validity score of the first bioelectric signal is determined as 40.

When the number of valid sampling points is between 200 and 400, the validity score of the first bioelectric signal is determined as 60.

When the number of valid sampling points is between 400 and 600, the validity score of the first bioelectric signal is determined as 80.

In some other optional embodiments, the doctor can estimate the strength of the first bioelectric signal according to the waveform of the first bioelectric signal, thereby determining whether the first bioelectric signal meets the preset condition.

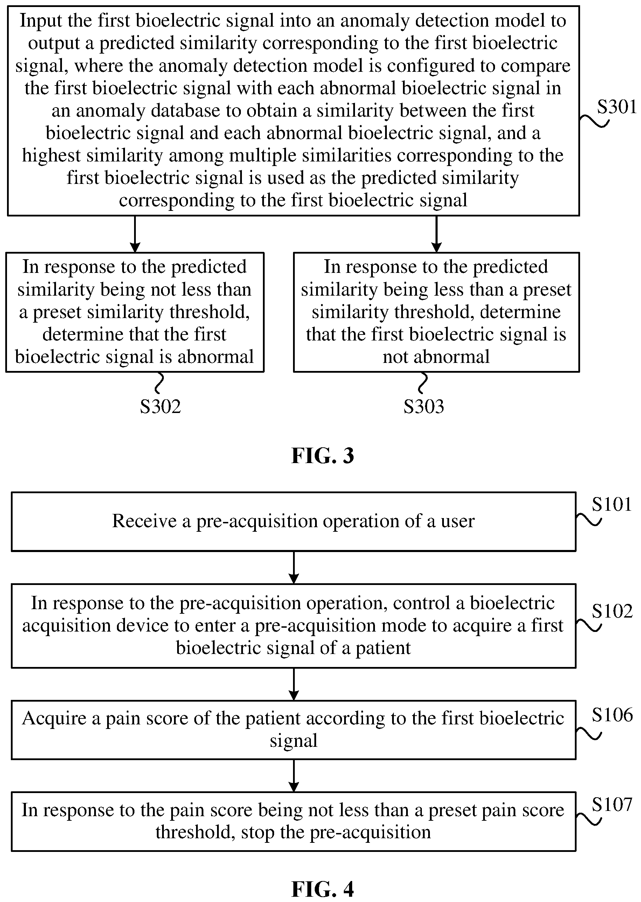

Referring to FIG. 3, FIG. 3 is a flowchart for detecting whether an abnormality has occurred according to an embodiment of the present application.

In some possible embodiments, a process of detecting whether the first bioelectric signal is abnormal may include steps S301 to S303.

In step S301, the first bioelectric signal is input into an anomaly detection model to output a predicted similarity corresponding to the first bioelectric signal, where the anomaly detection model is configured to compare the first bioelectric signal with each abnormal bioelectric signal in an anomaly database to obtain a similarity between the first bioelectric signal and each abnormal bioelectric signal, and a highest similarity among multiple similarities corresponding to the first bioelectric signal is used as the predicted similarity corresponding to the first bioelectric signal.

In step S302, in response to the predicted similarity being not less than a preset similarity threshold, that the first bioelectric signal is abnormal is determined.

In step S303, in response to the predicted similarity being less than a preset similarity threshold, that the first bioelectric signal is not abnormal is determined.

In this manner, the first bioelectric signal is input into the anomaly detection model, and the anomaly detection model is used for determining the predicted similarity corresponding to the first bioelectric signal. The anomaly detection model can call the anomaly database storing a large number of abnormal bioelectric signals which may be, specifically, bioelectric signals corresponding to different patients at the time of suffering from attacks or the bioelectric signals corresponding to the current patient at the time of suffering from the attack. When the current patient suffers from Parkinson's disease, the abnormal bioelectric signals in the anomaly database may include bioelectric signals corresponding to the Parkinson's patient at the time of suffering from the attack. On the one hand, the first bioelectric signal is compared with each abnormal bioelectric signal in the anomaly database in conjunction with a disease type to which an abnormal bioelectric signal with a highest similarity belongs to determine whether the patient suffers from the attack. On the other hand, the predicted similarity is compared with the preset similarity threshold to determine whether the first bioelectric signal is abnormal. Apparently, the higher the predicted similarity is, the more likely the first bioelectric signal is to be abnormal, and the more likely the patient is to suffer from the attack. The first bioelectric signal is compared with multiple abnormal bioelectric signals stored in the anomaly database one by one to find the abnormal bioelectric signal with the highest similarity, thereby further improving the accuracy of abnormal detection results.

The predicted similarity may be represented by a number or a percentage. The predicted similarity is, for example, 60, 80 or 90 when represented by a number and is, for example, 60%, 80% or 90% when represented by a percentage. The higher the value is, the higher the predicted similarity is.

The preset similarity threshold is not limited in the present application and may be 70%, 80% or 90%.

A training process of the anomaly detection model includes the steps described below.

A second training set is acquired, where the second training set includes multiple second training data, and each of the multiple second training data includes a first sample signal and a second sample signal that are used for training and annotated data of a similarity between the first sample signal and the second sample signal.

For each of the multiple second training data in the second training set, the processing described below is performed.

The first sample signal and the second sample signal in the second training data are input into a preset second deep learning model to obtain predicted data of the similarity between the first sample signal and the second sample signal.

A model parameter of the second deep learning model is updated according to the predicted data and the annotated data of the similarity between the first sample signal and the second sample signal.

Whether a preset second training termination condition is met is detected. In response to the preset second training termination condition being met, the trained second deep learning model is used as the anomaly detection model. In response to the preset second training termination condition not being met, the second deep learning model is continuously trained with the next second training data of the multiple second training data.

The preset second training termination condition is not limited in the present application and may be, for example, that the number of training reaches a preset number (the preset number is, for example, 1, 3, 10, 100, 1000 or 10000), all training data in the second training set are trained one or more times or a total loss value obtained after the training is not greater than a preset loss value.

In some possible embodiments, the preset condition includes: a validity score of the first bioelectric signal is not less than a preset validity score threshold.

In response to the first bioelectric signal not meeting the preset condition, the method may further include the steps described below.

Acquisition parameter configuration information corresponding to the bioelectric acquisition device is acquired according to the first bioelectric signal, and the bioelectric acquisition device is controlled to reenter the pre-acquisition mode.

In this manner, when the first bioelectric signal does not meet the preset condition, that is, when the validity score of the first bioelectric signal is less than the preset validity score threshold, it indicates that the signal strength of the first bioelectric signal is relatively low and the current acquisition parameter is inappropriate. The acquisition parameter configuration information can be acquired according to the first bioelectric signal to adjust the acquisition parameter and perform pre-acquisition again. The stepwise adjustment enables the pre-acquired bioelectric signal to meet the preset condition, thereby providing a reference basis for the subsequent formal acquisition.

The acquisition parameter configuration information is not limited in the present application and is, for example, a sampling bit of 10 bits and a data transmission rate of 1 Mbps.

Referring to FIG. 4, FIG. 4 is a portion of a flowchart of a method for controlling a programmed control device according to an embodiment of the present application.

In some possible embodiments, the first bioelectric signal includes an electroencephalogram signal, and after the bioelectric acquisition device is controlled to enter the pre-acquisition mode, the method may further include steps S106 and S 107.

In step S106, a pain score of the patient is acquired according to the first bioelectric signal.

In step S107, in response to the pain score being not less than a preset pain score threshold, the pre-acquisition is stopped.

In this manner, in the process of acquiring the bioelectric signal, whether a physical condition of the patient can withstand the process also needs to be considered. The pain score of the patient can be acquired according to the first bioelectric signal (the electroencephalogram signal) obtained through the pre-acquisition, thereby reflecting a pain degree of the patient. When the pain score of the patient is not less than the preset pain score threshold, it indicates that the patient is suffering from an unbearable pain. The pre-acquisition can be automatically stopped to alleviate the pain of the patient. This manner fully respects an individual feeling of the patient, reflects humanized care and significantly reduces a mental burden of the patient during the pre-acquisition.

The pain score may be represented by a number, for example, 60, 80 or 90. The higher the value is, the higher the pain score is. The pain score may also be represented by a letter, for example, A, B, C or D. The higher the ranking of the letter is, the higher the pain score is.

The preset pain score threshold is not limited in the present application and is, for example, 70, 80 or 85.

In some possible embodiments, the programmed control device is further configured to acquire the pain score of the patient in the manner described below.

The first bioelectric signal is input into a pain prediction model to obtain the pain score of the patient.

A training process of the pain prediction model includes the steps described below.

A first training set is acquired, where the first training set includes multiple first training data, and each of the multiple first training data includes a bioelectric signal of a sample object and annotated data of a pain score of the sample object.

For each of the multiple first training data in the first training set, the processing described below is performed.

The bioelectric signal of the sample object in each of the multiple first training data is input into a preset first deep learning model to obtain predicted data of the pain score of the sample object.

A model parameter of the first deep learning model is updated according to the predicted data and the annotated data of the pain score of the sample object.

Whether a preset first training termination condition is met is detected. In response to the preset first training termination condition being met, the trained first deep learning model is used as the pain prediction model. In response to the preset first training termination condition not being met, the first deep learning model is continuously trained with the next first training data of the multiple first training data.

In this manner, an appropriate number of neuron computing nodes and a multi-layer computational hierarchy are established through a design, and an appropriate input layer and output layer are selected to obtain the preset first deep learning model. A functional relationship between input and output is established through the learning and optimization of the preset first deep learning model. Although the functional relationship between input and output cannot be 100% found, a realistic correlation can be approached as close as possible. The pain prediction model trained in this manner can acquire the pain score of the patient according to the first bioelectric signal, and the accuracy and reliability of the calculation result are high.

A manner of acquiring the annotated data is not limited in the present application and may be, for example, a manual annotation manner, an automatic annotation manner or a semi-automatic annotation manner.

The training process of the pain prediction model is not limited in the present application and may be, for example, performed in a supervised learning training manner, a semi-supervised learning training manner or an unsupervised learning training manner.

The preset first training termination condition is not limited in the present application and may be, for example, that the number of training reaches a preset number (the preset number is, for example, 1, 3, 10, 100, 1000 or 10000), all training data in the first training set are trained one or more times or a total loss value obtained after the training is not greater than a preset loss value.

In some possible embodiments, the bioelectric acquisition device is a stimulator implanted into the patient, and the method may further include the step described below.

Stimulation parameter configuration information corresponding to the patient is acquired according to the second bioelectric signal after the formal acquisition is completed with the bioelectric acquisition device so that the bioelectric acquisition device applies corresponding electrical stimulation energy to the patient.

In this manner, the bioelectric acquisition device can have both functions of providing the electrical stimulation and sensing the bioelectric signal. The relatively long acquisition duration corresponding to the second bioelectric signal is very helpful for analyzing the condition of the patient. The stimulation parameter configuration information can be acquired according to the second bioelectric signal, and the bioelectric acquisition device can be used for providing accurate electrical stimulation to the patient.

The stimulation parameter configuration information is not limited in the present application and is, for example, a frequency of 130 Hz, a pulse width of 60 µs and an amplitude of 2-3 V

An embodiment of the present application further provides a programmed control device. Specific implementations of the programmed control device are the same as the implementations that are recorded in the above method embodiment and the technical effects that are achieved in the above method embodiment, and some contents are not repeated.

The programmed control device is configured to perform the operations described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

In some possible embodiments, the preset condition includes at least one of the conditions described below.

A validity score of the first bioelectric signal is not less than a preset validity score threshold.

The first bioelectric signal is not abnormal.

In some possible embodiments, the programmed control device is configured to acquire the validity score of the first bioelectric signal in the manners described below.

The first bioelectric signal is sampled to obtain multiple sampling points.

For each of the multiple sampling points, a comparison value between an amplitude of each of the multiple sampling points and a preset amplitude is calculated one by one, and a sampling point of the multiple sampling points whose comparison value is not less than a preset comparison threshold is used as a valid sampling point.

The validity score of the first bioelectric signal is determined according to the number of valid sampling points.

In some possible embodiments, the programmed control device is configured to detect whether the first bioelectric signal is abnormal in the manners described below.

The first bioelectric signal is input into an anomaly detection model to output a predicted similarity corresponding to the first bioelectric signal, where the anomaly detection model is configured to compare the first bioelectric signal with each abnormal bioelectric signal in an anomaly database to obtain a similarity between the first bioelectric signal and each abnormal bioelectric signal, and a highest similarity among multiple similarities corresponding to the first bioelectric signal is used as the predicted similarity corresponding to the first bioelectric signal.

In response to the predicted similarity being not less than a preset similarity threshold, that the first bioelectric signal is abnormal is determined.

In response to the predicted similarity being less than a preset similarity threshold, that the first bioelectric signal is not abnormal is determined.

In some possible embodiments, the preset condition includes: a validity score of the first bioelectric signal is not less than a preset validity score threshold.

In response to the first bioelectric signal not meeting the preset condition, the programmed control device is further configured to perform the operations described below.

Acquisition parameter configuration information corresponding to the bioelectric acquisition device is acquired according to the first bioelectric signal, and the bioelectric acquisition device is controlled to reenter the pre-acquisition mode.

In some possible embodiments, the first bioelectric signal includes an electroencephalogram signal, and after controlling the bioelectric acquisition device to enter the pre-acquisition mode, the programmed control device is further configured to perform the operations described below.

A pain score of the patient is acquired according to the first bioelectric signal.

In response to the pain score being not less than a preset pain score threshold, the pre-acquisition is stopped.

In some possible embodiments, the programmed control device is further configured to acquire the pain score of the patient in the manner described below.

The first bioelectric signal is input into a pain prediction model to obtain the pain score of the patient.

A training process of the pain prediction model includes the steps described below.

A first training set is acquired, where the first training set includes multiple first training data, and each of the multiple first training data includes a bioelectric signal of a sample object and annotated data of a pain score of the sample object.

For each of the multiple first training data in the first training set, the processing described below is performed.

The bioelectric signal of the sample object in each of the multiple first training data is input into a preset first deep learning model to obtain predicted data of the pain score of the sample object.

A model parameter of the first deep learning model is updated according to the predicted data and the annotated data of the pain score of the sample object.

Whether a preset first training termination condition is met is detected. In response to the preset first training termination condition being met, the trained first deep learning model is used as the pain prediction model. In response to the preset first training termination condition not being met, the first deep learning model is continuously trained with the next first training data of the multiple first training data.

In some possible embodiments, the bioelectric acquisition device is a stimulator implanted into the patient, and the programmed control device is further configured to perform the operation described below.

Stimulation parameter configuration information corresponding to the patient is acquired according to the second bioelectric signal after the formal acquisition is completed with the bioelectric acquisition device so that the bioelectric acquisition device applies corresponding electrical stimulation energy to the patient.

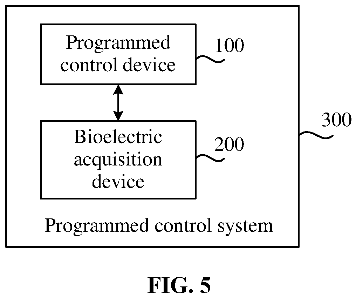

Referring to FIG. 5, FIG. 5 is a block diagram of a programmed control system 300 according to an embodiment of the present application.

An embodiment of the present application further provides a programmed control system 300. Specific implementations of the programmed control system 300 are the same as the implementations that are recorded in the above method embodiment and the technical effects that are achieved in the above method embodiment, and some contents are not repeated.

The programmed control system 300 includes a programmed control device 100 and a bioelectric acquisition device 200.

The programmed control device 100 is communicatively connected to the bioelectric acquisition device 200 and is configured to perform the operations described below.

A pre-acquisition operation of a user is received.

In response to the pre-acquisition operation, the bioelectric acquisition device 200 is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device 200 is controlled to enter a formal acquisition mode to acquire a second bioelectric signal of the patient, where an acquisition duration of the pre-acquisition mode is shorter than an acquisition duration of the formal acquisition mode.

An embodiment of the present application further provides an electronic device. The electronic device includes a memory and a processor, where the memory stores a computer program, and the processor, when executing the computer program, implements the steps described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

An embodiment of the present application further provides a computer-readable storage medium storing a computer program, where the computer program, when executed by a processor, implements the steps described below.

A pre-acquisition operation of a user is received.

In response to the pre-acquisition operation, a bioelectric acquisition device is controlled to enter a pre-acquisition mode to acquire a first bioelectric signal of a patient.

In response to the first bioelectric signal not meeting a preset condition, first prompt information is generated and sent to user equipment, and/or pre-acquisition is stopped.

In response to the first bioelectric signal meeting a preset condition, an acquisition confirmation operation of the user is received.

In response to the acquisition confirmation operation, the bioelectric acquisition device is controlled to enter a formal acquisition mode to acquire a second bioelectric signal of the patient, where an acquisition duration of the pre-acquisition mode is shorter than an acquisition duration of the formal acquisition mode.

It is to be noted that terms such as "first" and "second" in the description, claims and drawings of the present application are used to distinguish between similar objects and are not necessarily used to describe a particular order or sequence. It is to be understood that data used in this manner are interchangeable where appropriate so that the embodiments of the present application described herein can be implemented in an order not illustrated or described herein. Additionally, terms "include" and "have" and any variations thereof are intended to encompass a non-exclusive inclusion. For example, a process, method, system, product or device that includes a series of steps or units not only includes the expressly listed steps or units but may also include other steps or units that are not expressly listed or are inherent to such process, method, product or device.

When a component is referred to as being "on" or "above", "connected to" or "combined with" another component, the component may be directly on, directly connected to or directly combined with another component, or an intermediate component may exist. However, when a component is referred to as being "directly on", "directly connected to" or "directly combined with" another component, no intermediate component exists. Therefore, the term "connection" may refer to a physical connection or an electrical connection, and an intermediate component may exist or may not exist.

## Claims

1. A programmed control device configured to:
receive a pre-acquisition operation of a user;
in response to the pre-acquisition operation, control a bioelectric acquisition device to enter a pre-acquisition mode to acquire a first bioelectric signal of a patient;
in response to the first bioelectric signal not meeting a preset condition, perform at least one of: generating first prompt information and sending the first prompt information to user equipment, or stopping pre-acquisition;
in response to the first bioelectric signal meeting a preset condition, receive an acquisition confirmation operation of the user; and
in response to the acquisition confirmation operation, control the bioelectric acquisition device to enter a formal acquisition mode to acquire a second bioelectric signal of the patient, wherein an acquisition duration of the pre-acquisition mode is shorter than an acquisition duration of the formal acquisition mode.

2. The programmed control device according to claim 1, wherein the preset condition comprises at least one of the following:
a validity score of the first bioelectric signal is not less than a preset validity score threshold; or
the first bioelectric signal is not abnormal.

3. The programmed control device according to claim 2, wherein the programmed control device is configured to acquire the validity score of the first bioelectric signal in the following manners:
sampling the first bioelectric signal to obtain a plurality of sampling points;
for each of the plurality of sampling points, calculating a comparison value between an amplitude of each of the plurality of sampling points and a preset amplitude one by one, and using a sampling point of the plurality of sampling points whose comparison value is not less than a preset comparison threshold as a valid sampling point; and
determining the validity score of the first bioelectric signal according to a number of valid sampling points.

4. The programmed control device according to claim 2, wherein the programmed control device is configured to detect whether the first bioelectric signal is abnormal in the following manners:
inputting the first bioelectric signal into an anomaly detection model to output a predicted similarity corresponding to the first bioelectric signal, wherein the anomaly detection model is configured to compare the first bioelectric signal with each abnormal bioelectric signal in an anomaly database to obtain a similarity between the first bioelectric signal and each abnormal bioelectric signal, and a highest similarity among a plurality of similarities corresponding to the first bioelectric signal is used as the predicted similarity corresponding to the first bioelectric signal;
in response to the predicted similarity being not less than a preset similarity threshold, determining that the first bioelectric signal is abnormal; or
in response to the predicted similarity being less than a preset similarity threshold, determining that the first bioelectric signal is not abnormal.

5. The programmed control device according to claim 1, wherein the preset condition comprises:
a validity score of the first bioelectric signal is not less than a preset validity score threshold;
in response to the first bioelectric signal not meeting the preset condition, the programmed control device is further configured to:
acquire acquisition parameter configuration information corresponding to the bioelectric acquisition device according to the first bioelectric signal and control the bioelectric acquisition device to reenter the pre-acquisition mode.

6. The programmed control device according to claim 1, wherein the first bioelectric signal comprises an electroencephalogram signal, and after controlling the bioelectric acquisition device to enter the pre-acquisition mode, the programmed control device is further configured to:
acquire a pain score of the patient according to the first bioelectric signal; and
in response to the pain score being not less than a preset pain score threshold, stop the pre-acquisition.

7. The programmed control device according to claim 6, wherein the programmed control device is further configured to acquire the pain score of the patient in the following manner:
inputting the first bioelectric signal into a pain prediction model to obtain the pain score of the patient;
wherein a training process of the pain prediction model comprises:
acquiring a first training set, wherein the first training set comprises a plurality of first training data, and each of the plurality of first training data comprises a bioelectric signal of a sample object and annotated data of a pain score of the sample object;
for each of the plurality of first training data in the first training set, performing the following processing:
inputting the bioelectric signal of the sample object in each of the plurality of first training data into a preset first deep learning model to obtain predicted data of the pain score of the sample object;
updating a model parameter of the first deep learning model according to the predicted data and the annotated data of the pain score of the sample object; and
detecting whether a preset first training termination condition is met; in response to the preset first training termination condition being met, using the trained first deep learning model as the pain prediction model; in response to the preset first training termination condition not being met, continuously training the first deep learning model with a next first training data of the plurality of first training data.

8. The programmed control device according to claim 1, wherein the bioelectric acquisition device is a stimulator implanted into the patient, and the programmed control device is further configured to:
acquire stimulation parameter configuration information corresponding to the patient according to the second bioelectric signal after completing the formal acquisition with the bioelectric acquisition device so that the bioelectric acquisition device applies corresponding electrical stimulation energy to the patient.

9. A programmed control system, comprising a programmed control device and a bioelectric acquisition device;
wherein the programmed control device is communicatively connected to the bioelectric acquisition device and is configured to:
receive a pre-acquisition operation of a user;
in response to the pre-acquisition operation, control a bioelectric acquisition device to enter a pre-acquisition mode to acquire a first bioelectric signal of a patient;
in response to the first bioelectric signal not meeting a preset condition, perform at least one of: generating first prompt information and sending the first prompt information to user equipment, or stopping pre-acquisition;
in response to the first bioelectric signal meeting a preset condition, receive an acquisition confirmation operation of the user; and
in response to the acquisition confirmation operation, control the bioelectric acquisition device to enter a formal acquisition mode to acquire a second bioelectric signal of the patient, wherein an acquisition duration of the pre-acquisition mode is shorter than an acquisition duration of the formal acquisition mode.

10. An electronic device, comprising a memory and a processor, wherein the memory stores a computer program, and the processor, when executing the computer program, implements the following steps:
receiving a pre-acquisition operation of a user;
in response to the pre-acquisition operation, controlling a bioelectric acquisition device to enter a pre-acquisition mode to acquire a first bioelectric signal of a patient;
in response to the first bioelectric signal not meeting a preset condition, performing at least one of: generating first prompt information and sending the first prompt information to user equipment, or stopping pre-acquisition;
in response to the first bioelectric signal meeting a preset condition, receiving an acquisition confirmation operation of the user; and
in response to the acquisition confirmation operation, controlling the bioelectric acquisition device to enter a formal acquisition mode to acquire a second bioelectric signal of the patient, wherein an acquisition duration of the pre-acquisition mode is shorter than an acquisition duration of the formal acquisition mode.

11. A computer-readable storage medium storing a computer program, wherein the computer program, when executed by a processor, implements the following steps:
receiving a pre-acquisition operation of a user;
in response to the pre-acquisition operation, controlling a bioelectric acquisition device to enter a pre-acquisition mode to acquire a first bioelectric signal of a patient;
in response to the first bioelectric signal not meeting a preset condition, perform at least one of: generating first prompt information and sending the first prompt information to user equipment, or stopping pre-acquisition;
in response to the first bioelectric signal meeting a preset condition, receiving an acquisition confirmation operation of the user; and
in response to the acquisition confirmation operation, controlling the bioelectric acquisition device to enter a formal acquisition mode to acquire a second bioelectric signal of the patient, wherein an acquisition duration of the pre-acquisition mode is shorter than an acquisition duration of the formal acquisition mode.
